# EUROPÄISCHE PATENTSCHRIFT

(11) **EP 3 875 689 B1**
(45) Veröffentlichungstag und Bekanntmachung des Hinweises auf die Patenterteilung: **26.07.2023**
(21) Anmeldenummer: 20161283.5
(22) Anmeldetag: 05.03.2020
(51) Int. Cl.: E01H 1/08, A01G 20/47

(54) **HANDGEFÜHRTES SAUGSYSTEM**
HAND-HELD VACUUM SYSTEM
SYSTÈME D'ASPIRATION GUIDÉ À LA MAIN

(43) Veröffentlichungstag der Anmeldung: 08.09.2021
(73) Patentinhaber: Andreas Stihl AG & Co. KG, 71336 Waiblingen (DE)
(72) Erfinder: Pfeifer, Markus, 71364 Winnenden (DE); Seiz, Jonathan, 70376 Stuttgart (DE); Fuchs, Alexander, 74321 Bietigheim-Bissingen (DE); Oesterle, Markus, 71566 Althütte (DE)
(74) Vertreter: Patentanwälte Ruff, Wilhelm, Beier, Dauster & Partner mbB

(56) Entgegenhaltungen:
- EP-A1- 2 617 281
- EP-A1- 2 792 231
- US-A- 4 403 371

## Beschreibung

Die Erfindung bezieht sich auf ein handgeführtes Saugsystem.

Die EP 2 792 231 A1 offenbart ein Anbauteil für ein Blas/Saug-Gerät umfassend einen Sammelbeutel; einen Kuppler zum abnehmbaren Kuppeln des Anbauteils an einen Luftauslass des Blas/Saug-Geräts und zum Leiten von Luft von dem Luftauslass in den Sammelbeutel; eine drehbare Manschette, die an dem Sammelbeutel befestigt ist und drehbar an dem Kuppler angebracht ist, so dass der Sammelbeutel bezüglich des Luftauslasses drehbar ist.

### AUFGABE UND LÖSUNG

Der Erfindung liegt als Aufgabe die Bereitstellung eines handgeführten Saugsystems zugrunde, das verbesserte Eigenschaften aufweist, insbesondere benutzerfreundlicher ist.

Die Erfindung löst diese Aufgabe durch die Bereitstellung eines handgeführten Saugsystems mit den Merkmalen des Anspruchs 1. Vorteilhafte Weiterbildungen und/oder Ausgestaltungen der Erfindung sind in den abhängigen Ansprüchen beschrieben.

Das erfindungsgemäße handgeführte Saugsystem umfasst bzw. weist ein handgeführtes Sauggerät, eine Rohreinrichtung und eine Sackeinrichtung auf. Die Rohreinrichtung umfasst bzw. weist ein Saugrohr und, insbesondere nur, ein, insbesondere einziges, Rohrverbindungsteil auf. Die Sackeinrichtung umfasst bzw. weist einen Fangsack und, insbesondere nur, ein, insbesondere einziges, Sackverbindungsteil auf. Das Rohrverbindungsteil und das Sackverbindungsteil sind, insbesondere nur, zu einer, insbesondere einzigen und/oder unmittelbaren, mechanischen, insbesondere formschlüssigen, Verbindung miteinander zum Halten der Sackeinrichtung, insbesondere unmittelbar, durch die Rohreinrichtung ausgebildet bzw. konfiguriert. Das Rohrverbindungsteil ist an einem Umfang des Saugrohrs in Umfangsrichtung in einer oberen bzw. bodenabgewandten Hälfte des Umfangs in einer bestimmungsgemäßen Betriebsstellung bzw. -ausrichtung des Saugsystems derart, insbesondere vollständig, angeordnet und das Saugsystem ist derart ausgebildet bzw. konfiguriert, dass die durch die Rohreinrichtung gehaltene Sackeinrichtung, insbesondere mit einem Abschnitt, an, insbesondere in und/oder von, der oberen Hälfte des Umfangs in Umfangsrichtung nach unten an dem Saugrohr entlang verläuft, insbesondere in der bestimmungsgemäßen Betriebsstellung des Saugsystems.

Dies ermöglicht, dass die Sackeinrichtung, insbesondere der Fangsack, einem Benutzer des Saugsystems weniger oder nicht im Weg sein kann, insbesondere ist, insbesondere unterhalb des Saugrohrs und/oder des Sauggeräts, insbesondere im Unterschied zu einer, insbesondere vollständigen, Anordnung eines Rohrverbindungsteils an einem Umfang eines Saugrohrs in Umfangsrichtung an einem untersten Punkt des Umfangs in einer Betriebsstellung eines nicht-erfindungsgemäßen Saugsystems, insbesondere derart, dass eine durch eine Rohreinrichtung gehaltene Sackeinrichtung von dem untersten Punkt des Umfangs von dem Saugrohr nach unten weg verläuft.

Zusätzlich oder alternativ ermöglicht dies, dass der Fangsack ein großes Volumen, insbesondere für Sauggut, aufweisen bzw. haben kann, insbesondere aufweist bzw. hat, insbesondere im Unterschied zu dem nicht-erfindungsgemäßen Saugsystem.

Somit ermöglicht dies eine hohe Benutzerfreundlichkeit, insbesondere Benutzerergonomie, des Saugsystems.

Insbesondere kann das handgeführte (Englisch: hand-held) Saugsystem ein handgetragenes (Englisch: hand-portable) Saugsystem sein und/oder das handgeführte (Englisch: hand-held) Sauggerät kann ein handgetragenes (Englisch: hand-portable) Sauggerät sein. Zusätzlich oder alternativ kann handgeführtes, insbesondere handgetragenes, Saugsystem und/oder handgeführtes, insbesondere handgetragenes, Sauggerät bedeuten, dass das Saugsystem und/oder das Sauggerät eine Masse von maximal 50 Kilogramm (kg), insbesondere von maximal 20 kg, insbesondere von maximal 10 kg, insbesondere von maximal 5 kg, und/oder von minimal 1 kg, insbesondere von minimal 2 kg, aufweisen kann, insbesondere ohne Sauggut.

Das Saugsystem kann ein Laubsaugsystem sein und/oder das Sauggerät kann ein Laubsauggerät sein. Zusätzlich oder alternativ kann das Sauggerät ein Saughäcksler sein. Weiter zusätzlich oder alternativ kann das Sauggerät ein Lüftergebläse aufweisen und/oder zur Erzeugung eines Gas-Stroms, insbesondere eines Luft-Stroms, zur Bewegung von Sauggut in das Saugrohr hinein und durch das Saugrohr hindurch in den Fangsack hinein ausgebildet sein.

Die Rohreinrichtung und das Sauggerät können, insbesondere in einem bestimmungsgemäßen Betrieb des Saugsystems, aneinander für einen Sauggut-Strom von dem Saugrohr zu dem Sauggerät angeordnet sein, insbesondere miteinander mechanisch verbunden sein, insbesondere unmittelbar. Zusätzlich oder alternativ können die Rohreinrichtung und das Sauggerät, insbesondere in dem bestimmungsgemäßen Betrieb des Saugsystems, zum Halten der Rohreinrichtung, insbesondere unmittelbar, durch das Sauggerät miteinander mechanisch verbunden sein, insbesondere unmittelbar.

Die Sackeinrichtung und das Sauggerät können, insbesondere in einem, insbesondere dem, bestimmungsgemäßen Betrieb des Saugsystems, aneinander für einen Sauggut-Strom von dem Sauggerät zu dem Fangsack angeordnet sein, insbesondere miteinander mechanisch verbunden sein, insbesondere unmittelbar. Zusätzlich oder alternativ können die Sackeinrichtung und das Sauggerät, insbesondere in dem bestimmungsgemäßen Betrieb des Saugsystems, zum Halten der Sackeinrichtung, insbesondere unmittelbar, durch das Sauggerät miteinander mechanisch verbunden sein, insbesondere unmittelbar.

Das Sauggerät, die Rohreinrichtung und/oder die Sackeinrichtung können/kann, insbesondere jeweils, einstückig ausgebildet sein.

Das Saugrohr und das Rohrverbindungsteil können miteinander, insbesondere unmittelbar, mechanisch verbunden sein.

Der Fangsack und das Sackverbindungsteil können miteinander, insbesondere unmittelbar, mechanisch verbunden sein.

Der Umfang kann ein Außenumfang sein.

Die obere Hälfte kann in Umfangsrichtung auf halbem Weg und/oder halber Höhe von unten nach oben beginnen. Zusätzlich oder alternativ kann die obere Hälfte nach oben in der Betriebsstellung des Saugsystems gewandt sein.

Die Sackeinrichtung kann den Umfang, insbesondere nur, in seiner oberen Hälfte berühren.

Die bestimmungsgemäße Betriebsstellung kann durch die Schwerkraft bzw. die Gravitationskraft definiert sein.

Das Rohrverbindungsteil, insbesondere in der bestimmungsgemäßen Betriebsstellung des Saugsystems, ist derart angeordnet und das Saugsystem ist derart ausgebildet bzw. konfiguriert, dass der Fangsack, insbesondere mit einem Abschnitt, an der oberen Hälfte des Umfangs in Umfangsrichtung nach unten an dem Saugrohr entlang verläuft, insbesondere in der bestimmungsgemäßen Betriebsstellung des Saugsystems. Dies ermöglicht, dass der Fangsack ein besonders großes Volumen aufweisen bzw. haben kann, insbesondere aufweist bzw. hat.

In einer Weiterbildung der Erfindung verläuft der Fangsack in Strömungsrichtung minimal 0,2-mal, insbesondere minimal 0,3-mal, insbesondere minimal 0,4-mal, einer Länge des Saugrohrs in Strömungsrichtung an dem Saugrohr entlang.

Dies ermöglicht, dass der Fangsack ein besonders großes Volumen aufweisen bzw. haben kann, insbesondere aufweist bzw. hat. Zusätzlich oder alternativ ermöglicht dies ein gutes, insbesondere ausgerichtetes Halten, der Sackeinrichtung, insbesondere des Fangsacks.

Zusätzlich oder alternativ verläuft die Verbindung in Strömungsrichtung minimal 0,1-mal, insbesondere minimal 0,15-mal, insbesondere minimal 0,2-mal, einer, insbesondere der, Länge des Saugrohrs in Strömungsrichtung an dem Saugrohr entlang.

Dies ermöglicht ein gutes, insbesondere ausgerichtetes Halten, der Sackeinrichtung, insbesondere des Fangsacks.

Weiter zusätzlich oder alternativ ist das Rohrverbindungsteil von einem geräteseitigen Ende des Saugrohrs entgegen Strömungsrichtung minimal 0,2-mal, insbesondere 0,3-mal, insbesondere 0,4-mal, einer, insbesondere der, Länge des Saugrohrs in Strömungsrichtung, insbesondere angeordnet und/oder erstreckt.

Dies ermöglicht, dass der Fangsack ein besonders großes Volumen aufweisen bzw. haben kann, insbesondere aufweist bzw. hat.

Insbesondere kann der Fangsack in Strömungsrichtung maximal 1-mal, insbesondere maximal 0,8-mal, insbesondere maximal 0,6-mal, der Länge des Saugrohrs in Strömungsrichtung an dem Saugrohr entlang verlaufen. Zusätzlich oder alternativ kann die Verbindung in Strömungsrichtung maximal 1-mal, insbesondere maximal 0,6-mal, insbesondere maximal 0,4-mal, der Länge des Saugrohrs in Strömungsrichtung an dem Saugrohr entlang verlaufen.

Weiter zusätzlich oder alternativ kann das Rohrverbindungsteil von dem geräteseitigen Ende des Saugrohrs entgegen Strömungsrichtung maximal 1-mal, insbesondere maximal 0,8-mal, insbesondere maximal 0,6-mal, der Länge des Saugrohrs in Strömungsrichtung sein. Weiter zusätzlich oder alternativ kann die Strömungsrichtung eines Sauggut-Stroms und/oder eine Längsrichtung des Saugrohrs sein. Weiter zusätzlich oder alternativ kann das Saugrohr mit dem geräteseitigen Ende an dem Sauggerät in einem, insbesondere dem, bestimmungsgemäßen Betrieb des Saugsystems angeordnet sein.

In einer Weiterbildung der Erfindung sind das Rohrverbindungsteil und das Sackverbindungsteil zu der Verbindung miteinander zu einem, insbesondere durch den Benutzer, wahlweisen Verlauf der Sackeinrichtung, insbesondere mit einem Abschnitt, in Strömungsrichtung an, insbesondere entweder, einer linken Hälfte oder einer rechten Hälfte des Umfangs in der Betriebsstellung des Saugsystems ausgebildet bzw. konfiguriert. Dies ermöglicht, dass die Sackeinrichtung, insbesondere der Fangsack, dem Benutzer das Saugsystem führend wahlweise mit seiner rechten Hand oder seiner linken Hand weniger oder nicht im Weg sein kann, insbesondere ist. Insbesondere können das Rohrverbindungsteil und das Sackverbindungsteil zu der Verbindung miteinander zu dem wahlweisen Verlauf der Sackeinrichtung in Strömungsrichtung, insbesondere und in Umfangsrichtung, an einem linken, oberen Viertel oder einem rechten, oberen Viertel des Umfangs in der Betriebsstellung des Saugsystems ausgebildet sein. Zusätzlich oder alternativ kann die linke Hälfte in Umfangsrichtung auf halbem Weg und/oder halber Breite von rechts nach links beginnen. Weiter zusätzlich oder alternativ kann die linke Hälfte nach links in der Betriebsstellung des Saugsystems gewandt sein. Weiter zusätzlich oder alternativ kann die rechte Hälfte in Umfangsrichtung auf halbem Weg und/oder halber Breite von links nach rechts beginnen. Weiter zusätzlich oder alternativ kann die rechte Hälfte nach rechts in der Betriebsstellung des Saugsystems gewandt sein. Weiter zusätzlich oder alternativ kann die linke Hälfte auf einer Seite einer von oben nach unten und von vorne nach hinten, insbesondere in Strömungsrichtung und/oder durch eine Längsachse des Saugrohrs, verlaufenden Ebene in der Betriebsstellung des Saugsystems sein und die rechte Hälfte kann auf einer gegenüberliegenden Seite der Ebene sein. Weiter zusätzlich oder alternativ kann die Sackeinrichtung den Umfang, insbesondere nur, in seiner, entweder linken, Hälfte oder rechten Hälfte, insbesondere in seinem linken, oberen Viertel oder seinem rechten, oberen Viertel, berühren.

In einer Weiterbildung der Erfindung ist das Rohrverbindungsteil in Umfangsrichtung an einem obersten Punkt des Umfangs in der Betriebsstellung des Saugsystems, insbesondere vollständig, angeordnet. Dies ermöglicht den wahlweisen Verlauf der Sackeinrichtung in Strömungsrichtung an der linken Hälfte oder der rechten Hälfte des Umfangs in der Betriebsstellung des Saugsystems.

In einer Weiterbildung der Erfindung umfasst bzw. weist das Rohrverbindungsteil eine Schlaufenaufhängung auf, insbesondere ist das Rohrverbindungsteil eine Schlaufenaufhängung. Zusätzlich oder alternativ umfasst bzw. weist das Sackverbindungsteil eine Schlaufe auf, insbesondere ist das Sackverbindungsteil eine Schlaufe. Dies ermöglicht eine einfache Herstellung der Verbindung. Zusätzlich oder alternativ ermöglicht dies den wahlweisen Verlauf der Sackeinrichtung in Strömungsrichtung an der linken Hälfte oder der rechten Hälfte des Umfangs in der Betriebsstellung des Saugsystems. Insbesondere kann die Schlaufe mit ihren Enden an dem Fangsack angeordnet sein.

In einer Weiterbildung der Erfindung ist das Saugsystem zu einer, insbesondere werkzeugfrei und/oder zerstörungsfrei, lösbaren Anordnung, insbesondere mechanischen Verbindung, der Sackeinrichtung an, insbesondere mit, dem Sauggerät und der Rohreinrichtung ausgebildet bzw. konfiguriert. Zusätzlich oder alternativ ist das Saugsystem zu einer, insbesondere werkzeugfrei und/oder zerstörungsfrei, lösbaren Anordnung, insbesondere mechanischen Verbindung, der Rohreinrichtung an, insbesondere mit, dem Sauggerät und der Sackeinrichtung ausgebildet bzw. konfiguriert. Dies ermöglicht ein einfaches Transportieren und/oder platzsparendes Lagern der Sackeinrichtung und/oder der Rohreinrichtung, insbesondere des Saugsystems. Zusätzlich oder alternativ ermöglicht dies ein einfaches Entleeren der Sackeinrichtung, insbesondere des Fangsacks. Insbesondere können/kann die Sackeinrichtung und/oder die Rohreinrichtung, insbesondere jeweils, als Anbauteil bezeichnet werden.

In einer Ausgestaltung der Erfindung ist die Schlaufe als Tragegriff zum Tragen der gelösten Sackeinrichtung durch einen, insbesondere den, Benutzer ausgebildet bzw. konfiguriert. Dies ermöglicht eine Multifunktion der Schlaufe.

In einer Ausgestaltung der Erfindung umfasst bzw. weist der Fangsack eine wiederverschließbare Entleerungsöffnung, insbesondere zum Entleeren von Sauggut, auf. Die Schlaufe und die Entleerungsöffnung sind derart zueinander angeordnet, dass beim und/oder durch Tragen der gelösten Sackeinrichtung, insbesondere nur, an der Schlaufe der Fangsack mit der Entleerungsöffnung nicht nach unten ausgerichtet bzw. gewandt ist. Dies ermöglicht ein Risiko einer unbeabsichtigten Entleerung des Fangsacks beim Tragen zu vermeiden oder sogar zu verhindern. Insbesondere kann beim Tragen der Fangsack mit der Entleerungsöffnung nach oben ausgerichtet sein. Zusätzlich oder alternativ kann beim Tragen der Fangsack durch die Schwerkraft bzw. die Gravitationskraft ausgerichtet sein.

In einer Weiterbildung der Erfindung umfasst bzw. weist der Fangsack eine, insbesondere die und/oder mittels eines Reißverschlusses, wiederverschließbare Entleerungsöffnung, insbesondere zum Entleeren von Sauggut, auf. Der Fangsack umfasst bzw. weist mindestens in einem Endbereich der Entleerungsöffnung eine Hinterlegung auf. Dies ermöglicht ein Risiko einer unbeabsichtigten Entleerung des Fangsacks bei nicht vollständig verschlossener Entleerungsöffnung zu vermeiden oder sogar zu verhindern. Insbesondere kann die Hinterlegung eine Dichtlage, insbesondere aus Textil, aufweisen, insbesondere sein.

In einer Weiterbildung der Erfindung ist eine Form des Fangsacks gegenüber einer, insbesondere beliebigen bzw. frei wählbaren, von oben nach unten und von vorne nach hinten, insbesondere in Strömungsrichtung, verlaufenden Ebene in der Betriebsstellung des Saugsystems nicht spiegelsymmetrisch, insbesondere unabhängig von dem wahlweisen Verlauf der Sackeinrichtung in Strömungsrichtung an der linken Hälfte oder der rechten Hälfte des Umfangs in der Betriebsstellung des Saugsystems, soweit vorhanden.

Dies ermöglicht, dass die Sackeinrichtung, insbesondere der Fangsack, dem Benutzer das Saugsystem führend mit, insbesondere entweder, seiner rechten Hand oder seiner linken Hand weniger oder nicht im Weg sein kann, insbesondere ist.

Zusätzlich oder alternativ ist eine Gasdurchlässigkeit des Fangsacks gegenüber einer, insbesondere beliebigen bzw. frei wählbaren und/oder der, von oben nach unten und von vorne nach hinten, insbesondere in Strömungsrichtung, verlaufenden Ebene in der Betriebsstellung des Saugsystems nicht spiegelsymmetrisch, insbesondere unabhängig von dem wahlweisen Verlauf der Sackeinrichtung in Strömungsrichtung an der linken Hälfte oder der rechten Hälfte des Umfangs in der Betriebsstellung des Saugsystems, soweit vorhanden.

Dies ermöglicht, dass der Benutzer das Saugsystem führend mit, insbesondere entweder, seiner rechten Hand oder seiner linken Hand weniger oder nicht angeblasen werden kann, insbesondere wird.

Insbesondere kann der Fangsack ein gasdurchlässiges Textil aufweisen.

In einer Weiterbildung der Erfindung ist die Rohreinrichtung zu einer, insbesondere der, Anordnung an einer Rohrseite des Sauggeräts, insbesondere an dem Sauggerät und/oder vollständig, ausgebildet bzw. konfiguriert, insbesondere angeordnet. Die Sackeinrichtung ist zu einer, insbesondere der, Anordnung an einer von der Rohrseite verschiedenen, insbesondere abgewandten, Sackseite des Sauggeräts, insbesondere an dem Sauggerät und/oder mindestens teilweise, ausgebildet bzw. konfiguriert, insbesondere angeordnet. Dies ermöglicht eine gute Balance des Saugsystems und/oder einen guten Strömungsverlauf in dem Sauggerät. Insbesondere kann ein Winkel zwischen einer Orthogonalen bzw. einer Normalen der Rohrseite und einer Orthogonalen bzw. einer Normalen der Sackseite minimal 75 Grad (°) und/oder maximal 165 °, insbesondere 90 ° sein. Zusätzlich oder alternativ kann das Rohrverbindungsteil an einer der Sackseite bzw. der Anordnung der Sackeinrichtung an dem Sauggerät abgewandten, insbesondere gegenüberliegenden, Seite des Umfangs des Saugrohrs angeordnet sein.

In einer Ausgestaltung der Erfindung ist die Rohrseite, insbesondere mit einer Orthogonalen bzw. Normalen, nach vorne unten, insbesondere entgegen Strömungsrichtung, in der Betriebsstellung des Saugsystems gewandt bzw. ausgerichtet. Die Saugseite ist, insbesondere mit einer Orthogonalen bzw. Normalen, nach hinten, insbesondere und unten, in der Betriebsstellung des Saugsystems gewandt bzw. ausgerichtet.

In einer Weiterbildung der Erfindung umfasst bzw. weist das Sauggerät, insbesondere mindestens, einen Handgriff für einen, insbesondere den, Benutzer zum Führen, insbesondere und zum Tragen, des Saugsystems auf. Der Handgriff ist in der Betriebsstellung des Saugsystems oben an dem Sauggerät, insbesondere vollständig, angeordnet. Dies ermöglicht ein einfaches Führen, insbesondere Tragen, des Saugsystems. Insbesondere kann beim und/oder durch Tragen des Sauggeräts, insbesondere an dem mindestens einen Handgriff, insbesondere nur an dem mindestens einen Handgriff, das Saugsystem in die Betriebsstellung, insbesondere durch die Schwerkraft bzw. die Gravitationskraft, ausgerichtet sein.

In einer Weiterbildung der Erfindung hat bzw. weist das Saugrohr an dem Rohrverbindungsteil einen runden, insbesondere kreisrunden, Querschnitt auf. Dies ermöglicht eine einfache Herstellung des Saugrohrs und/oder einen geringen Strömungswiderstand des Saugrohrs. Insbesondere kann der Querschnitt orthogonal bzw. normal zur Strömungsrichtung sein. Zusätzlich oder alternativ kann das Saugrohr einen elliptischen Querschnitt aufweisen bzw. haben.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind. Dabei zeigen:
- Fig. 1: eine Perspektivansicht eines erfindungsgemäßem handgeführten Saugsystems von vorne rechts,
- Fig. 2: eine weitere Perspektivansicht des Saugsystems der Fig. 1 von vorne links,
- Fig. 3: eine weitere Perspektivansicht des Saugsystems der Fig. 1 von rechts,
- Fig. 4: eine weitere Perspektivansicht des Saugsystems der Fig. 1 von hinten,
- Fig. 5: eine Ansicht einer Sackeinrichtung des Saugsystems der Fig. 1 beim Tragen,
- Fig. 6: eine Ansicht einer Hinterlegung einer Entleerungsöffnung eines Fangsacks der Sackeinrichtung der Fig. 5,
- Fig. 7: eine Querschnittansicht einer Rohreinrichtung des Saugsystems der Fig. 1, und
- Fig. 8: eine Ansicht eines Sauggeräts des Saugsystems der Fig. 1.

### DETAILLIERTE BESCHREIBUNG DER AUSFÜHRUNGSBEISPIELE

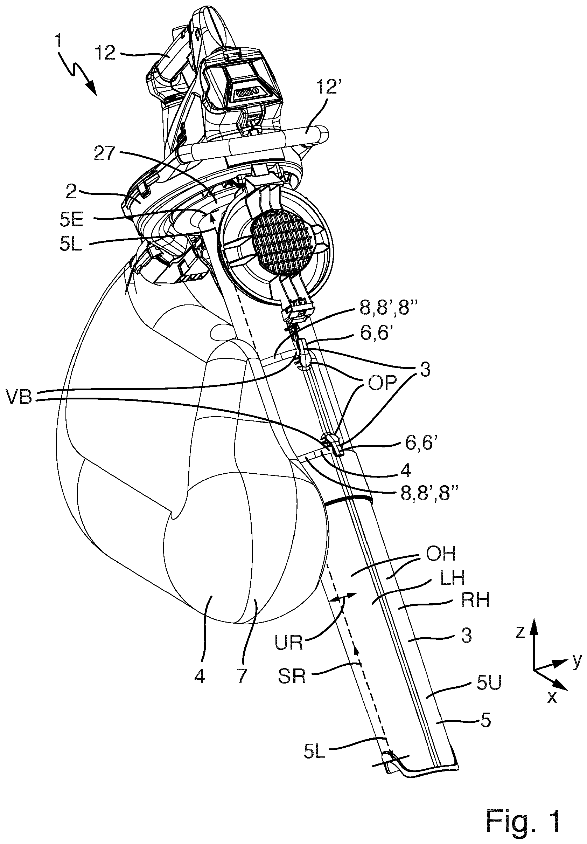

Fig. 1 bis 4 zeigen ein erfindungsgemäßes handgeführtes Saugsystem 1. Das Saugsystem 1 weist ein handgeführtes Sauggerät 2, eine Rohreinrichtung 3 und eine Sackeinrichtung 4 auf. Die Rohreinrichtung 3 weist ein Saugrohr 5 und ein Rohrverbindungsteil 6 auf. Die Sackeinrichtung 4 weist einen Fangsack 7 und ein Sackverbindungsteil 8 auf. Das Rohrverbindungsteil 6 und das Sackverbindungsteil 8 sind zu einer mechanischen Verbindung VB miteinander zum Halten der Sackeinrichtung 4 durch die Rohreinrichtung 3 ausgebildet, insbesondere sind das Rohrverbindungsteil 6 und das Sackverbindungsteil 8 miteinander mechanisch verbunden. Das Rohrverbindungsteil 6 ist an einem Umfang 5U des Saugrohrs 5 in Umfangsrichtung UR in einer oberen Hälfte OH des Umfangs 5U in einer bestimmungsgemäßen Betriebsstellung des Saugsystems 1 derart angeordnet und das Saugsystem 1 ist derart ausgebildet, dass die durch die Rohreinrichtung 3 gehaltene Sackeinrichtung 4 an der oberen Hälfte OH des Umfangs 5U in Umfangsrichtung UR nach unten an dem Saugrohr 5 entlang verläuft.

Im Detail ist das Rohrverbindungsteil 6 derart angeordnet und das Saugsystem 1 ist derart ausgebildet, dass der Fangsack 7, insbesondere an der oberen Hälfte OH des Umfangs 5U in Umfangsrichtung UR nach unten, an dem Saugrohr 5 entlang verläuft, wie in Fig. 1 gezeigt.

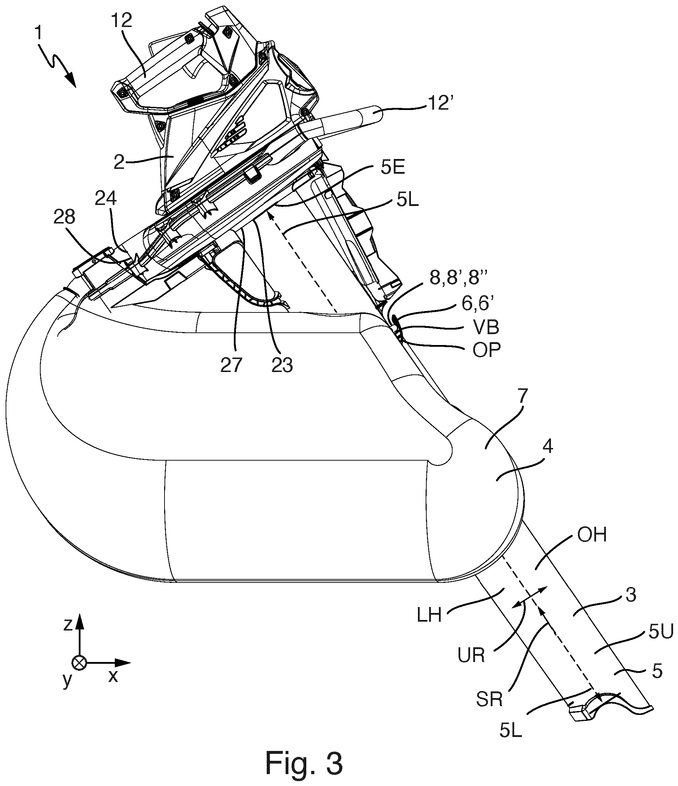

Des Weiteren verläuft der Fangsack 7 in Strömungsrichtung SR, insbesondere in -xz-Richtung, minimal 0,2-mal, insbesondere minimal 0,3-mal, insbesondere minimal 0,4-mal, einer Länge 5L des Saugrohrs 5 in Strömungsrichtung SR an dem Saugrohr 5 entlang, wie in Fig. 3 gezeigt.

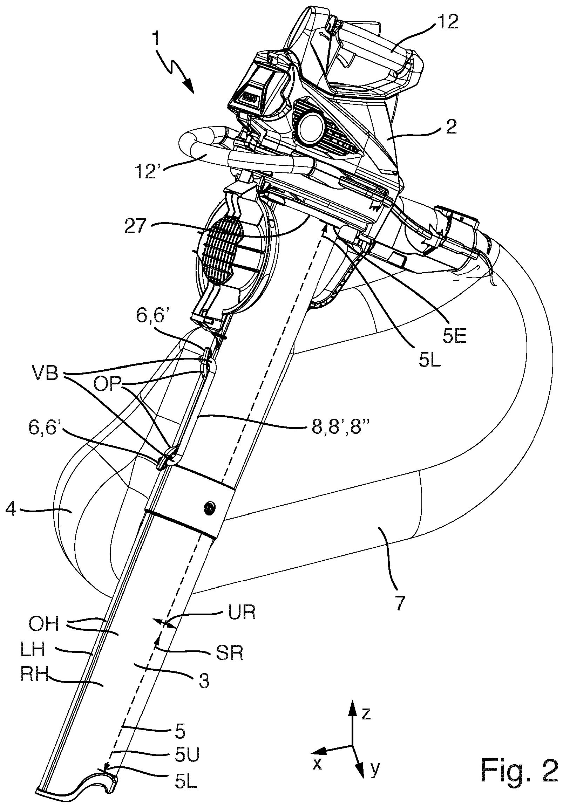

Zusätzlich verläuft die Verbindung VB in Strömungsrichtung SR minimal 0,1-mal, insbesondere minimal 0,15-mal, insbesondere minimal 0,2-mal, der Länge 5L des Saugrohrs 5 in Strömungsrichtung SR an dem Saugrohr 5 entlang, wie in Fig. 1 und 2 gezeigt.

Weiter zusätzlich ist das Rohrverbindungsteil 6 von einem geräteseitigen Ende 5E des Saugrohrs 5 entgegen Strömungsrichtung SR minimal 0,2-mal, insbesondere 0,3-mal, insbesondere 0,4-mal, der Länge 5L des Saugrohrs 5 in Strömungsrichtung SR, insbesondere angeordnet und/oder erstreckt, wie in Fig. 1 bis 3 gezeigt.

Außerdem sind das Rohrverbindungsteil 6 und das Sackverbindungsteil 8 zu der Verbindung VB miteinander zu einem wahlweisen Verlauf der Sackeinrichtung 4 in Strömungsrichtung SR an einer linken Hälfte LH, wie in Fig. 1 bis 4 gezeigt, oder einer rechten Hälfte RH des Umfangs 5 in der Betriebsstellung des Saugsystems 1 ausgebildet.

Weiter ist das Rohrverbindungsteil 6 in Umfangsrichtung UR an einem obersten Punkt OP des Umfangs 5 in der Betriebsstellung des Saugsystems 1 angeordnet.

Zudem weist das Rohrverbindungsteil 6 eine Schlaufenaufhängung 6' auf, insbesondere ist das Rohrverbindungsteil 6 eine Schlaufenaufhängung 6`.

Zusätzlich weist das Sackverbindungsteil 8 eine Schlaufe 8' auf, insbesondere ist das Sackverbindungsteil 8 eine Schlaufe 8`.

In anderen Worten: das Rohrverbindungsteil 6 und das Sackverbindungsteil 8 sind so, dass das Sackverbindungsteil 8 asymmetrisch an dem symmetrischen Rohrverbindungsteil 6 aufgehängt ist.

Des Weiteren ist das Saugsystem 1 zu einer, insbesondere werkzeugfrei, lösbaren Anordnung der Sackeinrichtung 4 an dem Sauggerät 2 und der Rohreinrichtung 3 ausgebildet.

Zusätzlich ist das Saugsystem 1 zu einer, insbesondere werkzeugfrei, lösbaren Anordnung der Rohreinrichtung 3 an dem Sauggerät 2 und der Sackeinrichtung 4 ausgebildet.

In Fig. 1 bis 4 ist die Sackeinrichtung 4 an dem Sauggerät 2 und der Rohreinrichtung 3 angeordnet. Zusätzlich ist die Rohreinrichtung 3 an dem Sauggerät 2 und der Sackeinrichtung 4 angeordnet.

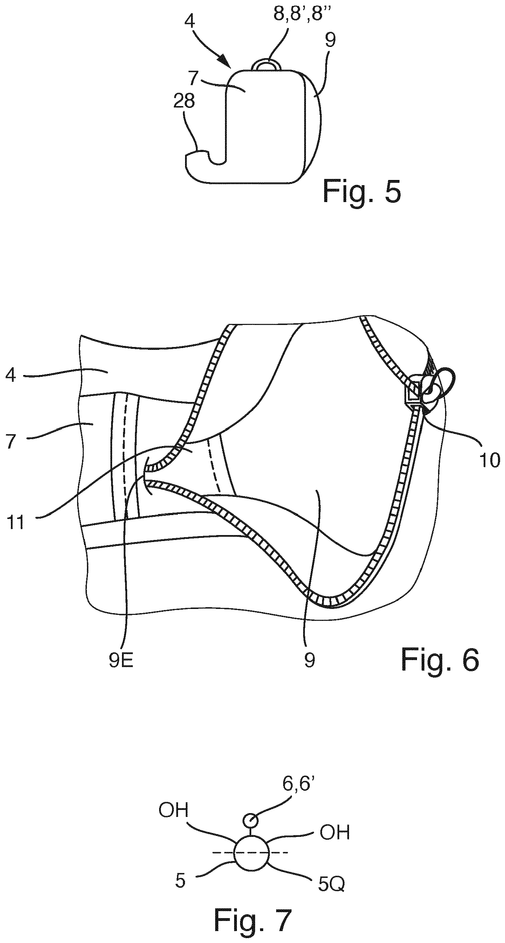

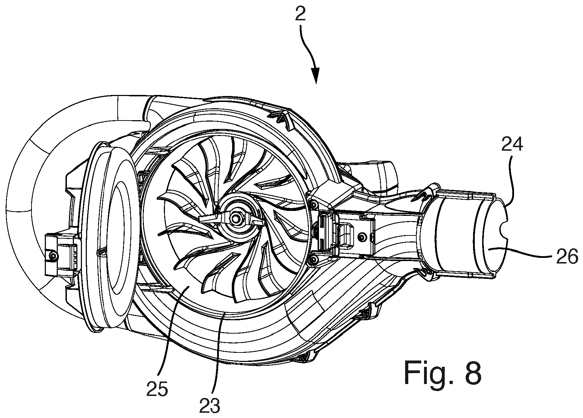

In Fig. 5 und 8 ist die Sackeinrichtung 4 von dem Sauggerät 2 und der Rohreinrichtung 3 gelöst.

In Fig. 7 und 8 ist die Rohreinrichtung 3 von dem Sauggerät 2 und der Sackeinrichtung 4 gelöst.

Im Detail ist die Schlaufe 8' als Tragegriff 8" zum Tragen der gelösten Sackeinrichtung 4 durch einen Benutzer ausgebildet.

Außerdem weist der Fangsack 7 eine, insbesondere mittels eines Reißverschlusses 10, wiederverschließbare Entleerungsöffnung 9 auf, wie in Fig. 5 und 6 gezeigt.

Die Schlaufe 8' und die Entleerungsöffnung 9 sind derart zueinander angeordnet, dass beim Tragen der gelösten Sackeinrichtung 4 an der Schlaufe 8' der Fangsack 7 mit der Entleerungsöffnung 9 nicht nach unten ausgerichtet ist, wie in Fig. 5 gezeigt.

Weiter weist der Fangsack 7 mindestens in einem Endbereich 9E der Entleerungsöffnung 9 eine Hinterlegung 11 auf, wie in Fig. 6 gezeigt.

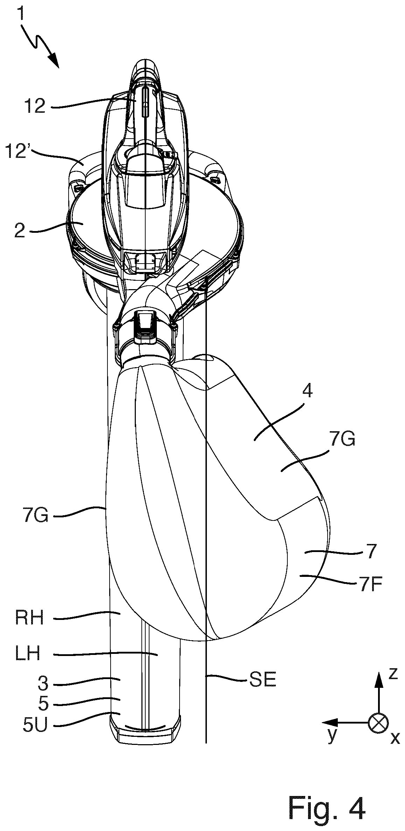

Zudem ist eine Form 7F des Fangsacks 7 gegenüber einer von oben nach unten, insbesondere in -z-Richtung, und von vorne nach hinten, insbesondere in -x-Richtung, verlaufenden Ebene SE in der Betriebsstellung des Saugsystems 1 nicht spiegelsymmetrisch, wie in Fig. 4 gezeigt. In anderen Worten: der Fangsack 7 ist asymmetrisch ausgeführt.

In dem gezeigten Ausführungsbeispiel ermöglicht dies, dass die Sackeinrichtung 4, insbesondere der Fangsack 7, dem Benutzer das Saugsystem 1 führend mit seiner rechten Hand weniger oder nicht im Weg sein kann, insbesondere ist.

In alternativen Ausführungsbeispielen kann die Form des Fangsacks gegenüber der von oben nach unten und von vorne nach hinten verlaufenden Ebene in der Betriebsstellung des Saugsystems nicht spiegelsymmetrisch sein, so dass dies ermöglichen kann, dass die Sackeinrichtung, insbesondere der Fangsack, dem Benutzer das Saugsystem führend mit seiner linken Hand weniger oder nicht im Weg sein kann, insbesondere ist.

Zusätzlich ist eine Gasdurchlässigkeit 7G des Fangsacks 7 gegenüber der von oben nach unten und von vorne nach hinten verlaufenden Ebene SE in der Betriebsstellung des Saugsystems 1 nicht spiegelsymmetrisch, wie in Fig. 4 gezeigt.

In dem gezeigten Ausführungsbeispiel ermöglicht dies, dass der Benutzer das Saugsystem 1 führend mit seiner rechten Hand weniger oder nicht angeblasen werden kann, insbesondere wird. In anderen Worten: die Gasdurchlässigkeit 7G des Fangsacks 7 ist in -y-Richtung größer als in y-Richtung. Nochmals in anderen Worten: gasdurchlässige Bereiche sind so am Fangsack 7 angebracht, dass Gas hauptsächlich vom Benutzer weggeleitet wird.

In alternativen Ausführungsbeispielen kann die Gasdurchlässigkeit des Fangsacks gegenüber der von oben nach unten und von vorne nach hinten verlaufenden Ebene in der Betriebsstellung des Saugsystems nicht spiegelsymmetrisch sein, so dass dies ermöglichen kann, dass der Benutzer das Saugsystem führend mit seiner linken Hand weniger oder nicht angeblasen werden kann, insbesondere wird.

Des Weiteren ist die Rohreinrichtung 3 zu der Anordnung an einer Rohrseite 23 des Sauggeräts 2 ausgebildet, insbesondere angeordnet. Die Sackeinrichtung 4 ist zu der Anordnung an einer von der Rohrseite 23 verschiedenen, insbesondere abgewandten, Sackseite 24 des Sauggeräts 2 ausgebildet, insbesondere angeordnet.

Im Detail ist die Rohrseite 23 nach vorne unten, insbesondere in der x-z-Richtung, in der Betriebsstellung des Saugsystems 1 gewandt, wie in Fig. 1 bis 4 gezeigt. Die Saugseite 24 ist nach hinten, insbesondere in der -x-Richtung, insbesondere und unten, insbesondere in der -z-Richtung, in der Betriebsstellung des Saugsystems 1 gewandt.

Insbesondere weist das Sauggerät 2 eine Rohrverbindungsöffnung 25 auf, wie in Fig. 8 gezeigt. Die Rohreinrichtung 3, insbesondere das Saugrohr 5, weist eine Rohrgeräteverbindungsöffnung 27 auf. Die Rohreinrichtung 3 ist mit der Rohrgeräteverbindungsöffnung 27 zu der Anordnung an der Rohrverbindungsöffnung 25 des Sauggeräts 2 ausgebildet, insbesondere angeordnet.

Außerdem weist das Sauggerät 2 eine Sackverbindungsöffnung 26 auf, wie in Fig. 8 gezeigt. Die Sackeinrichtung 4, insbesondere der Fangsack 7, weist eine Sackgeräteverbindungsöffnung 28 auf. Die Sackeinrichtung 4 ist mit der Sackgeräteverbindungsöffnung 28 zu der Anordnung an der Sackverbindungsöffnung 26 des Sauggeräts 2 ausgebildet, insbesondere angeordnet.

Weiter weist das Sauggerät 2 einen Handgriff 12 für den Benutzer zum Führen des Saugsystems 1 auf. Der Handgriff 12 ist in der Betriebsstellung des Saugsystems 1 oben an dem Sauggerät 2 angeordnet.

In dem gezeigten Ausführungsbeispiel weist das Sauggerät 2 einen weiteren Handgriff 12' auf.

Zudem weist das Saugrohr 5 an dem Rohrverbindungsteil 6 einen runden, insbesondere kreisrunden, Querschnitt 5Q auf, wie in Fig. 7 gezeigt.

Wie die gezeigten und zuvor beschriebenen Ausführungsbeispiele deutlich machen, stellt die Erfindung ein handgeführtes Saugsystem bereit, das verbesserte Eigenschaften aufweist, insbesondere benutzerfreundlicher ist.

## Patentansprüche

1. Handgeführtes Saugsystem (1), wobei das Saugsystem (1) aufweist:
- ein handgeführtes Sauggerät (2), eine Rohreinrichtung (3) und eine Sackeinrichtung (4),
- wobei die Rohreinrichtung (3) ein Saugrohr (5) und ein Rohrverbindungsteil (6) aufweist,
- wobei die Sackeinrichtung (4) einen Fangsack (7) und ein Sackverbindungsteil (8) aufweist, und
- wobei das Rohrverbindungsteil (6) und das Sackverbindungsteil (8) zu einer mechanischen Verbindung (VB) miteinander zum Halten der Sackeinrichtung (4) durch die Rohreinrichtung (3) ausgebildet sind,
**dadurch gekennzeichnet,**
- **dass** das Rohrverbindungsteil (6) an einem Umfang (5U) des Saugrohrs (5) in Umfangsrichtung (UR) in einer oberen Hälfte (OH) des Umfangs (5U) in einer bestimmungsgemäßen Betriebsstellung des Saugsystems (1) derart angeordnet ist und das Saugsystem (1) derart ausgebildet ist, dass die durch die Rohreinrichtung (3) gehaltene Sackeinrichtung (4) an der oberen Hälfte (OH) des Umfangs (5U) in Umfangsrichtung (UR) nach unten an dem Saugrohr (5) entlang verläuft, und
- **dass** das Rohrverbindungsteil (6) derart angeordnet ist und das Saugsystem (1) derart ausgebildet ist, dass der Fangsack (7) an der oberen Hälfte (OH) des Umfangs (5U) in Umfangsrichtung (UR) nach unten an dem Saugrohr (5) entlang verläuft.

2. Handgeführtes Saugsystem (1) nach dem vorhergehenden Anspruch,
- wobei der Fangsack (7) in Strömungsrichtung (SR) minimal 0,2-mal, insbesondere minimal 0,3-mal, insbesondere minimal 0,4-mal, einer Länge (5L) des Saugrohrs (5) in Strömungsrichtung (SR) an dem Saugrohr (5) entlang verläuft, und/oder
- wobei die Verbindung (VB) in Strömungsrichtung (SR) minimal 0,1-mal, insbesondere minimal 0,15-mal, insbesondere minimal 0,2-mal, einer Länge (5L) des Saugrohrs (5) in Strömungsrichtung (SR) an dem Saugrohr (5) entlang verläuft, und/oder
- wobei das Rohrverbindungsteil (6) von einem geräteseitigen Ende (5E) des Saugrohrs (5) entgegen Strömungsrichtung (SR) minimal 0,2-mal, insbesondere 0,3-mal, insbesondere 0,4-mal, einer Länge (5L) des Saugrohrs (5) in Strömungsrichtung (SR) ist.

3. Handgeführtes Saugsystem (1) nach einem der vorhergehenden Ansprüche,
- wobei das Rohrverbindungsteil (6) und das Sackverbindungsteil (8) zu der Verbindung (VB) miteinander zu einem wahlweisen Verlauf der Sackeinrichtung (4) in Strömungsrichtung (SR) an einer linken Hälfte (LH) oder einer rechten Hälfte (RH) des Umfangs (5) in der Betriebsstellung des Saugsystems (1) ausgebildet sind.

4. Handgeführtes Saugsystem (1) nach einem der vorhergehenden Ansprüche,
- wobei das Rohrverbindungsteil (6) in Umfangsrichtung (UR) an einem obersten Punkt (OP) des Umfangs (5) in der Betriebsstellung des Saugsystems (1) angeordnet ist.

5. Handgeführtes Saugsystem (1) nach einem der vorhergehenden Ansprüche,
- wobei das Rohrverbindungsteil (6) eine Schlaufenaufhängung (6') aufweist, insbesondere ist, und/oder
- wobei das Sackverbindungsteil (8) eine Schlaufe (8`) aufweist, insbesondere ist.

6. Handgeführtes Saugsystem (1) nach einem der vorhergehenden Ansprüche,
- wobei das Saugsystem (1) zu einer, insbesondere werkzeugfrei, lösbaren Anordnung der Sackeinrichtung (4) an dem Sauggerät (2) und der Rohreinrichtung (3) ausgebildet ist, und/oder
- wobei das Saugsystem (1) zu einer, insbesondere werkzeugfrei, lösbaren Anordnung der Rohreinrichtung (3) an dem Sauggerät (2) und der Sackeinrichtung (4) ausgebildet ist.

7. Handgeführtes Saugsystem (1) nach Ansprüchen 5 und 6,
- wobei die Schlaufe (8`) als Tragegriff (8") zum Tragen der gelösten Sackeinrichtung (4) durch einen Benutzer ausgebildet ist.

8. Handgeführtes Saugsystem (1) nach Anspruch 7,
- wobei der Fangsack (7) eine wiederverschließbare Entleerungsöffnung (9) aufweist, wobei die Schlaufe (8`) und die Entleerungsöffnung (9) derart zueinander angeordnet sind, dass beim Tragen der gelösten Sackeinrichtung (4) an der Schlaufe (8`) der Fangsack (7) mit der Entleerungsöffnung (9) nicht nach unten ausgerichtet ist.

9. Handgeführtes Saugsystem (1) nach einem der vorhergehenden Ansprüche,
- wobei der Fangsack (7) eine, insbesondere mittels eines Reißverschlusses (10), wiederverschließbare Entleerungsöffnung (9) aufweist, wobei der Fangsack (7) mindestens in einem Endbereich (9E) der Entleerungsöffnung (9) eine Hinterlegung (11) aufweist.

10. Handgeführtes Saugsystem (1) nach einem der vorhergehenden Ansprüche,
- wobei eine Form (7F) des Fangsacks (7) gegenüber einer von oben nach unten und von vorne nach hinten verlaufenden Ebene (SE) in der Betriebsstellung des Saugsystems (1) nicht spiegelsymmetrisch ist, und/oder
- wobei eine Gasdurchlässigkeit (7G) des Fangsacks (7) gegenüber einer von oben nach unten und von vorne nach hinten verlaufenden Ebene (SE) in der Betriebsstellung des Saugsystems (1) nicht spiegelsymmetrisch ist.

11. Handgeführtes Saugsystem (1) nach einem der vorhergehenden Ansprüche,
- wobei die Rohreinrichtung (3) zu einer Anordnung an einer Rohrseite (23) des Sauggeräts (2) ausgebildet ist, insbesondere angeordnet ist, und
- wobei die Sackeinrichtung (4) zu einer Anordnung an einer von der Rohrseite (23) verschiedenen, insbesondere abgewandten, Sackseite (24) des Sauggeräts (2) ausgebildet ist, insbesondere angeordnet ist.

12. Handgeführtes Saugsystem (1) nach Anspruch 11,
- wobei die Rohrseite (23) nach vorne unten in der Betriebsstellung des Saugsystems (1) gewandt ist, und
- wobei die Saugseite (24) nach hinten, insbesondere und unten, in der Betriebsstellung des Saugsystems (1) gewandt ist.

13. Handgeführtes Saugsystem (1) nach einem der vorhergehenden Ansprüche,
- wobei das Sauggerät (2) einen Handgriff (12) für einen Benutzer zum Führen des Saugsystems (1) aufweist, wobei der Handgriff (12) in der Betriebsstellung des Saugsystems (1) oben an dem Sauggerät (2) angeordnet ist.

14. Handgeführtes Saugsystem (1) nach einem der vorhergehenden Ansprüche,
- wobei das Saugrohr (5) an dem Rohrverbindungsteil (6) einen runden, insbesondere kreisrunden, Querschnitt (5Q) aufweist.

## Claims

1. Hand-held suction system (1), wherein the suction system (1) has:
- a hand-held suction device (2), a tube unit (3) and a bag unit (4),
- wherein the tube unit (3) has a suction tube (5) and a tube connecting part (6),
- wherein the bag unit (4) has a collecting bag (7) and a bag connecting part (8), and
- wherein the tube connecting part (6) and the bag connecting part (8) are designed to form a mechanical connection (VB) to one another in order to hold the bag unit (4) by means of the tube unit (3),
**characterised in,**
- **that** the tube connecting part (6) is arranged in such a way on a circumference (5U) of the suction tube (5), in an upper half (OH) of the circumference (5U) in a circumferential direction (UR), in a correct operating position of the suction system (1), and the suction system (1) is designed in such a way that the bag unit (4) held by the tube unit (3) extends downwards in the circumferential direction (UR) along the suction tube (5), on the upper half (OH) of the circumference (5U), and
- **that** the tube connecting part (6) is arranged in such a way and the suction system (1) is designed in such a way that the collecting bag (7) extends along the suction tube (5) downwards in the circumferential direction (UR) on the upper half (OH) of the circumference (5U).

2. Hand-held suction system (1) according to the preceding claim,
- wherein, in the flow direction (SR), the collecting bag (7) extends along the suction tube (5) for a minimum of 0.2 times, in particular a minimum of 0.3 times, in particular a minimum of 0.4 times, a length (5L) of the suction tube (5) in the flow direction (SR), and/or
- wherein, in the flow direction (SR), the connection (VB) extends along the suction tube (5) for a minimum of 0.1 times, in particular a minimum of 0.15 times, in particular a minimum of 0.2 times, a length (5L) of the suction tube (5) in the flow direction (SR), and/or
- wherein, counter to the flow direction (SR), the tube connecting part (6) is a minimum of 0.2 times, in particular 0.3 times, in particular 0.4 times, a length (5L) of the suction tube (5) in the flow direction (SR) from a device-side end (5E) of the suction tube (5).

3. Hand-held suction system (1) according to any of the preceding claims,
- wherein the tube connecting part (6) and the bag connecting part (8) are designed to form the connection (VB) to one another so as to allow the bag unit (4) to run optionally along a left-hand half (LH) or a right-hand half (RH) of the circumference (5) in the flow direction (SR) in the operating position of the suction system (1).

4. Hand-held suction system (1) according to any of the preceding claims,
- wherein the tube connecting part (6) is arranged at an uppermost point (OP) of the circumference (5) in a circumferential direction (UR) in the operating position of the suction system (1).

5. Hand-held suction system (1) according to any of the preceding claims,
- wherein the tube connecting part (6) has, in particular is, a loop hanger arrangement (6'), and/or
- wherein the bag connecting part (8) has, in particular is, a loop (8').

6. Hand-held suction system (1) according to any of the preceding claims,
- wherein the suction system (1) is designed to form an arrangement of the bag unit (4) on the suction device (2) and the tube unit (3) which can be released, in particular without tools, and/or
- wherein the suction system (1) is designed to form an arrangement of the tube unit (3) on the suction device (2) and the bag unit (4) which can be released, in particular without tools.

7. Hand-held suction system (1) according to claims 5 and 6,
- wherein the loop (8') is designed as a carrying handle (8'') to enable the released bag unit (4) to be carried by a user.

8. Hand-held suction system (1) according to claim 7,
- wherein the collecting bag (7) has a resealable emptying aperture (9), wherein the loop (8') and the emptying aperture (9) are arranged in such a way relative to one another that the emptying aperture (9) of the collecting bag (7) is not oriented downwards when the released bag unit (4) is being carried by the loop (8').

9. Hand-held suction system (1) according to any of the preceding claims,
- wherein the collecting bag (7) has an emptying aperture (9) that can be resealed, in particular by means of a zip fastener (10), wherein the collecting bag (7) has a backing (11), at least in an end region (9E) of the emptying aperture (9).

10. Hand-held suction system (1) according to any of the preceding claims,
- wherein a shape (7F) of the collecting bag (7) is not mirror-symmetrical with respect to a plane (SE) extending from the top down and from the front to the rear in the operating position of the suction system (1), and/or
- wherein a gas permeability (7G) of the collecting bag (7) is not mirror-symmetrical with respect to a plane (SE) extending from the top down and from the front to the rear in the operating position of the suction system (1).

11. Hand-held suction system (1) according to any of the preceding claims,
- wherein the tube unit (3) is designed to form an arrangement, in particular is arranged, on a tube side (23) of the suction device (2), and
- wherein the bag unit (4) is designed to form an arrangement, in particular is arranged, on a bag side (24) of the suction device (2) which is different from the tube side (23), in particular a side facing away therefrom.

12. Hand-held suction system (1) according to claim 11,
- wherein the tube side (23) faces forwards and downwards in the operating position of the suction system (1), and
- wherein the suction side (24) faces rearwards, in particular and downwards, in the operating position of the suction system (1).

13. Hand-held suction system (1) according to any of the preceding claims,
- wherein the suction device (2) has a handle (12) for a user for the purpose of guiding the suction system (1), wherein the handle (12) is arranged on the top of the suction device (2) in the operating position of the suction system (1).

14. Hand-held suction system (1) according to any of the preceding claims,
- wherein the suction tube (5) has a round, in particular circular, cross section (5Q) at the tube connecting part (6).

## Revendications

1. Système d'aspiration manuel (1), le système d'aspiration (1) présentant :
- un appareil d'aspiration manuel (2), un dispositif de tuyau (3) et un dispositif de sac (4),
- le dispositif de tuyau (3) présentant un tuyau d'aspiration (5) et une partie de connexion de tuyau (6),
- le dispositif de sac (4) présentant un sac de collecte (7) et une partie de connexion de sac (8), et
- la partie de connexion de tuyau (6) et la partie de connexion de sac (8) étant configurées pour une connexion mécanique (VB) l'une à l'autre pour maintenir le dispositif de sac (4) à travers le dispositif de tuyau (3),
**caractérisé en ce que**
- la partie de connexion de tuyau (6) est agencée sur une circonférence (5U) du tuyau d'aspiration (5) dans la direction circonférentielle (UR) dans une moitié supérieure (OH) de la circonférence (5U) dans une position de fonctionnement prévue du système d'aspiration (1) et le système d'aspiration (1) est configuré de telle sorte que le dispositif de sac (4) maintenu par le dispositif de tuyau (3) s'étend sur la moitié supérieure (OH) de la circonférence (5U) dans la direction circonférentielle (UR) vers le bas le long du tuyau d'aspiration (5), et
- **en ce que** la partie de connexion de tuyau (6) est agencée et le système d'aspiration (1) est configuré de telle sorte que le sac de collecte (7) s'étend sur la moitié supérieure (OH) de la circonférence (5U) dans la direction circonférentielle (UR) vers le bas le long du tuyau d'aspiration (5).

2. Système d'aspiration manuel (1) selon la revendication précédente,
- le sac de collecte (7) s'étendant dans la direction d'écoulement (SR) sur au minimum 0,2 fois, notamment au minimum 0,3 fois, notamment au minimum 0,4 fois, une longueur (5L) du tuyau d'aspiration (5) dans la direction d'écoulement (SR) le long du tuyau d'aspiration (5), et/ou
- la connexion (VB) s'étendant dans la direction d'écoulement (SR) sur au minimum 0,1 fois, notamment au minimum 0,15 fois, notamment au minimum 0,2 fois, une longueur (5L) du tuyau d'aspiration (5) dans la direction d'écoulement (SR) le long du tuyau d'aspiration (5), et/ou
- la partie de connexion de tuyau (6) étant à partir d'une extrémité côté appareil (5E) du tuyau d'aspiration (5) à l'encontre de la direction d'écoulement (SR) à au minimum 0,2 fois, notamment 0,3 fois, notamment 0,4 fois, une longueur (5L) du tuyau d'aspiration (5) dans la direction d'écoulement (SR).

3. Système d'aspiration manuel (1) selon l'une quelconque des revendications précédentes,
- la partie de connexion de tuyau (6) et la partie de connexion de sac (8) étant configurées pour la connexion (VB) l'une à l'autre pour une extension sélective du dispositif de sac (4) dans la direction d'écoulement (SR) sur une moitié gauche (LH) ou une moitié droite (RH) de la circonférence (5) dans la position de fonctionnement du système d'aspiration (1).

4. Système d'aspiration manuel (1) selon l'une quelconque des revendications précédentes,
- la partie de connexion de tuyau (6) étant agencée dans la direction circonférentielle (UR) à un point supérieur (OP) de la circonférence (5) dans la position de fonctionnement du système d'aspiration (1).

5. Système d'aspiration manuel (1) selon l'une quelconque des revendications précédentes,
- la partie de connexion de tuyau (6) présentant, notamment étant, une suspension à boucle (6'), et/ou
- la partie de connexion de sac (8) présentant, notamment étant, une boucle (8').

6. Système d'aspiration manuel (1) selon l'une quelconque des revendications précédentes,
- le système d'aspiration (1) étant configuré pour un agencement détachable, notamment sans outil, du dispositif de sac (4) sur l'appareil d'aspiration (2) et le dispositif de tuyau (3), et/ou
- le système d'aspiration (1) étant configuré pour un agencement détachable, notamment sans outil, du dispositif de tuyau (3) sur l'appareil d'aspiration (2) et le dispositif de sac (4).

7. Système d'aspiration manuel (1) selon les revendications 5 et 6,
- la boucle (8') étant configurée sous forme de poignée de transport (8'') pour le transport du dispositif de sac détaché (4) par un utilisateur.

8. Système d'aspiration manuel (1) selon la revendication 7,
- le sac de collecte (7) présentant une ouverture de vidage refermable (9), la boucle (8') et l'ouverture de vidage (9) étant agencées l'une par rapport à l'autre de telle sorte que lorsque le dispositif de sac détaché (4) est transporté au niveau de la boucle (8'), le sac de collecte (7) n'est pas orienté avec l'ouverture de vidage (9) vers le bas.

9. Système d'aspiration manuel (1) selon l'une quelconque des revendications précédentes,
- le sac de collecte (7) présentant une ouverture de vidage (9) refermable, notamment au moyen d'une fermeture à glissière (10), le sac de collecte (7) présentant une contre-dépouille (11) au moins dans une zone d'extrémité (9E) de l'ouverture de vidage (9).

10. Système d'aspiration manuel (1) selon l'une quelconque des revendications précédentes,
- une forme (7F) du sac de collecte (7) n'étant pas symétrique par rapport à un plan (SE) s'étendant de haut en bas et d'avant en arrière dans la position de fonctionnement du système d'aspiration (1), et/ou
- un passage de gaz (7G) du sac de collecte (7) n'étant pas symétrique par rapport à un plan (SE) s'étendant de haut en bas et d'avant en arrière dans la position de fonctionnement du système d'aspiration (1).

11. Système d'aspiration manuel (1) selon l'une quelconque des revendications précédentes,
- le dispositif de tuyau (3) étant configuré, notamment agencé, pour un agencement sur un côté de tuyau (23) de l'appareil d'aspiration (2), et
- le dispositif de sac (4) étant configuré, notamment agencé, pour un agencement sur un côté de sac (24) de l'appareil d'aspiration (2) différent, notamment détourné, du côté de tuyau (23).

12. Système d'aspiration manuel (1) selon la revendication 11,
- le côté de tuyau (23) étant tourné vers l'avant et le bas dans la position de fonctionnement du système d'aspiration (1), et
- le côté d'aspiration (24) étant tourné vers l'arrière, notamment et vers le bas, dans la position de fonctionnement du système d'aspiration (1).

13. Système d'aspiration manuel (1) selon l'une quelconque des revendications précédentes,
- l'appareil d'aspiration (2) présentant une poignée (12) pour un utilisateur afin de guider le système d'aspiration (1), la poignée (12) étant agencée sur le dessus de l'appareil d'aspiration (2) dans la position de fonctionnement du système d'aspiration (1).

14. Système d'aspiration manuel (1) selon l'une quelconque des revendications précédentes,
- le tuyau d'aspiration (5) présentant une section transversale ronde, notamment circulaire (5Q), au niveau de la partie de connexion de tuyau (6).
